(19) Europäisches Patentamt
European Patent Office
Office européen des brevets

(11) EP 3 384 917 B1

(12) **EUROPEAN PATENT SPECIFICATION**

(45) Date of publication and mention of the grant of the patent:
**15.09.2021 Bulletin 2021/37**

(21) Application number: **16871127.3**

(22) Date of filing: **09.11.2016**

(51) Int Cl.:
***A61K 47/59*** *(2017.01)*
***A61K 31/787*** *(2006.01)*
***A61K 38/43*** *(2006.01)*
***A61P 19/04*** *(2006.01)*
***A61P 29/00*** *(2006.01)*

(86) International application number:
**PCT/RU2016/000755**

(87) International publication number:
**WO 2017/095264 (08.06.2017 Gazette 2017/23)**

(54) **METHOD FOR PRODUCING HYALURONIDASE CONJUGATE WITH POLYETHYLENEPIPERAZINE DERIVATIVES AND THE USE OF THE CONJUGATE PRODUCED**

VERFAHREN ZUR HERSTELLUNG EINES HYALURONIDASEKONJUGATS MIT POLYETHYLENPIPERAZINDERIVATEN UND VERWENDUNG DES HERGESTELLTEN KONJUGATS

PROCÉDÉ DE PRODUCTION DE CONJUGUÉ DE HYALURONIDASE AVEC DES DÉRIVÉS DE POLYÉTHYLÈNE PIPERAZINE ET UTILISATION DU CONJUGUÉ OBTENU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2015 RU 2015152036**

(43) Date of publication of application:
**10.10.2018 Bulletin 2018/41**

(73) Proprietor: **Obshchestvo S Ogranichennoy Otvetstvennostyu "NPO Petrovaks Farm"**
**Podolsk 142143 (RU)**

(72) Inventors:
- **NEKRASOV, Arkadii Vasilevich**
  **Moscow 121351 (RU)**
- **KARAPUTADZE, Temuri Musaevich**
  **Moscow 117113 (RU)**
- **MEDVEDEV, Sergei Alekseevich**
  **Moscow 117418 (RU)**
- **KOZUKOV, Alexander Vladimirovich**
  **Krasnogorsk 143400 (RU)**
- **KARAPUTADZE, Nino Temurievna**
  **Moscow 117113 (RU)**

(74) Representative: **Fortuna, Jevgenijs**
**Foral Patent Law Office**
**P.O. Box 98**
**1050 Riga (LV)**

(56) References cited:
**RU-C1- 2 112 542**
**RU-C2- 2 556 378**
**US-A- 5 811 100**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 384 917 B1

Printed by Jouve, 75001 PARIS (FR)

## Description

This invention relates to development of technologies for obtaining immobilized enzyme preparations. It can be widely used in various branches of industry, particularly in pharmaceutical industry, for producing stable, active and safe-in-application long-acting medicinal preparations (drugs).

The most effective method of stabilization of compounds of protein or other nature in a physiological solution consists in chemical conjugation based on high-molecular carriers [Parveen S, Sahoo S.K., Clin Pharmacokinet. 2006;45(10), p.965; Duncan R., PEGylated Protein Drugs: Basic Science and Clinical Applications. Ed.: Birkhäuser Basel 2009; Harris J.M, Chess RB. Effect of pegylation on pharmaceuticals. //Nat Rev Drug Discov, 2003, 2(3), p.214.

Covalent binding of medicinal proteins with a polymer carrier increases manifold conformational stability of protein molecules and their resistance to protease and specific inhibitors effect, which makes possible creation of micromolecular bioactive long-acting drugs based on them [Nekrasov A.V., Puchkova N.G., Immunology (Immunologiya), 2006; 27(2), p.1].

According to present knowledge, there are plenty polymer carriers used to prepare conjugates with protein and non-protein compounds. The most known are the conjugates based on polyethylene glycole (PEG), used as medicinal preparations [Beilon P.S., Palleroni A. B. /Interferon Conjugates (Conjugaty interferona), Patent RU 2180595. Burg Y, Hilger B., Josel H.-P., Patent RU 2232163; Kurochkin S.N., Parkansky A.A., Patent RU 2298560].

There are known water-soluble polymer heterocyclic amines, for example, derivatives of N-oxide poly-1,4-ethylene piperazine. Polymer amines are unique in their properties and applicability as a polymer carrier for conjugation. Copolymer of N-oxide 1,4-ethylene piperazine and (N-carboxymethyl)-1,4-ethylene piperazinium halogenide (Polyoxidonium) is non-toxic. It possesses antiradical and detoxification properties, and it is bio-degradable due to N-oxide groups. This copolymer is allowed for application in medical practice, and it is used as an immune-modulator, adjuvant or polymer carrier [Nekrasov A.V, Puchkova N.G., Ivanova A.S. Derivatives of poly-1,4-ethylene piperazine having immune-modulating, antivirus, antibacterial activities. Patent RU 2073031]. This makes urgent and important the directions of development of easy-to-reach and simple technologies of production of polymer carriers, compliance with all specified requirements, and various conjugation processes on their basis.

Patent RU 2185388 (published on 20.07.2002) describes oxidation of poly-1,4- ethylene piperazine in an aqueous solution containing a solvent and an oxidizer and being capable to produce atomic oxygen under normal conditions. It is appropriate to use as an oxidizer organic and inorganic peroxides and hydroperoxides, salts of oxygenated haloacids, ozone, oxygen, all resulting from water electrolysis.

There can be used as an acidic vehicle, for example, strong acetic acid water solution.

It is reasonable to perform oxidation by way of mixing poly-1,4-ethylene piperazine (PEP) with acetic acid (AA) aqueous solution and hydrogen peroxide (HP) in a molecular ratio: PEP:AA:HP=1:0.45:0.7. The oxidation process goes on in a heterogeneous medium until complete dissolution of the polymer is reached. Then the obtained N-oxide poly-1,4-ethylene piperazine is subjected to alkylation in the presence of an alkylating agent. This process is carried out in aqueous environment. It is recommended to use as an alkylating agent the substances coming in covalent bonding with a tertiary nitrogen atom in a polymer chain within a temperature range from 30 to 100 °C, for example, haloacids or their esters of cyclic or acyclic construction.

It is preferable to use bromoacetic acid ester as the alkylating agent.

The solution of copolymer of N-oxide poly-1,4- ethylene piperazine and (N-carboxyethyl)-1,4-ethylene piperazinium bromide (C-PNO) obtained in the process of alkylation, upon its cleaning, for example, by ultrafiltration, can be used for further synthesis of high-molecular water-soluble biogenic compounds, particularly conjugates or complexes of biologically active substances.

Patent RU 2556378 (published on 10.07.2015) describes the conjugate of glycoprotein having activity of erythropoietin and a method of its production. Said method includes oxidation of polyethylene piperazine to N-oxide, and alkylation by bromoacetic acid with resultant copolymer N-oxide poly-1,4- ethylene piperazine and (N-carboxymethyl)-1,4-ethylene piperazinium bromide, and cleaning of obtained copolymer. Copolymer conjugation with erythropoietin is carried out by carbodiimide or hydrazide method.

The closest to the claimed invention is the invention described in Patente RU 2112542 (published on 10.06.1998). This Patent relates to the drug comprising conjugate of hyaluronidase enzyme for treatment of pathological state of connective tissues. This Patente describes a method for obtaining a conjugate of hyaluronidase enzyme with high-molecular carrier - copolymer of N-oxide poly-1,4-ethylene piperazine and (N-carboxyethyl)-1,4-ethylene piperazinium bromide (Polyoxidonium), mol.wt. 40,000-100,000 D, and enzyme: carrier ratio of 1: (1-5) corresponding to the general formula

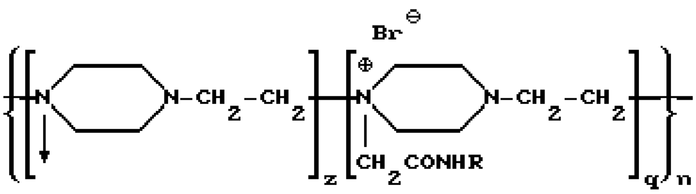

where R is the hyaluronidase enzyme; n = 300-700 is the number of unit units; q = 0.2-0.4 is the number of alkylated units; z = 0.4-0.8 is the number of oxygenated units. At that, hyaluronidase, segregated from bovine seminal glands, can be used as enzyme in the drug. This method consists in conjugation of hyaluronidase with Polyoxidonium using either an azide method or a method of activated succinimide esters. When using azide method the conjugate is obtained in two steps: at the first step Polyoxidonium hydrazide is obtained from Polyoxidonium, and at the second step the conjugate is obtained by way of reaction of Polyoxidonium azide coupling with an enzyme. When using the method of activated esters, first, a Polyoxidonium succinimide ester is obtained, and then it is conjugated with an enzyme. The conjugated drug was named as Longidaza. The method for obtaining copolymer of N-oxide poly-1,4-ethylene piperazine and (N-carboxyethyl)-1,4-ethylene piperazinium bromide is not described in this Patent.

Great demand for preparations having the properties of Longidaza drug at the pharmaceutical market, economic and environment requirements prompted improvement of the process used for production with a purpose of ruling out some disadvantages and gaining rise in productivity.

All above-mentioned Patents do not describe distribution of molecular units in the copolymer structure, or the conjugate obtained on its basis.

The studies revealed that distribution of molecular units in the copolymer structure exerts effect upon the properties of the conjugate produced, namely, on the degree of conjugation, outputs of target product and stability of conjugated preparations.

The present invention object consists in development of a method for getting a conjugate of hyaluronidase enzyme with a polymer carrier comprising the copolymer of N-oxide poly-1,4-ethylene piperazine and (N-carboxymethyl)-1,4-ethylene piperazinium bromide having at the same time the properties of inhibition of hyperplasia of connective tissue and anti-inflammatory action, being fit for treatment of pathological state of connective tissues with improved properties and increased method output.

The assigned task can be solved by a method for obtaining active conjugate of hyaluronidase enzyme with a copolymer comprising N-oxide 1,4-ethylene piperazine and (N-carboxymethyl)-1,4-ethylene piperazinium halogenide using carbodiimide or azide method for conjugation, cleaning and freeze drying. The conjugation is performed with the use of water-soluble copolymer, which is a copolymer of N-oxide 1,4-ethylene piperazine, (N-carboxymethyl)-1,4-ethylene piperazinium or its hydrazide derivative and 1,4-ethylene piperazine of general formula (I)

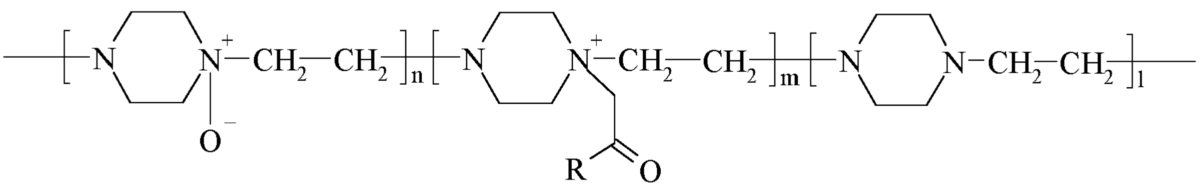

R = OH, $NHNH_2$

where n is ranging from 40 % to 90 % of total number of units;
m is ranging from 3 % to 40 % of total number of units;

$$n + m + l = 100\ \%,$$

obtained from poly-1,4-ethylene piperazine with the use of the step of oxidation by means of an oxidizing agent being able to generate atomic oxygen in the presence of urea, alkylation by means of lower haloacetic acid or its alkyl ester, and hydrazinolysis in the case of azide method.

The invention for which protection is sought refers only to methods for the preparation of an active conjugate of hyaluronidase enzyme with a copolymer as defined in claims 1-10 and to medical preparations as defined in claim 11.

Urea is added at the oxidation step in amount of 1 to 10 wt. %, preferably 3 to 6 wt.%, per total mass of reaction feed, water included.

The conjugation is carried out with the use of hyaluronidase taken from bovine seminal glands.

Cleaning is carried out by washing with purified water on semitransparent cassette with the lower limit of particles cutoff ranging from 1 to 30 kDa.

The copolymer used is a random polymer, the units of which in the molecule structure are in disordered state and can be in any order and combination.

The profile of distribution of functional groups influences the polymer carrier reactivity in conjugation reaction, as well as stability of copolymer and conjugates based on this copolymer.

The copolymer derived by using various methods and having the same quantity with similar active groups (N-oxide and carboxyl groups) demonstrates absolutely different reactivity in conjugation reaction. This is due to that carboxyl groups can be difficult to access because of steric reasons and/or not be activated when N-oxide groups are behind. Usage of a polymer carrier with low-reactive carboxyl groups leads to significant decrease of conjugation yield and extent, worsens molecular weight distribution of derived conjugates. Up to the present invention the heterocyclic N-oxide polymer carrier did not have the required number of reactive groups (carboxyl or hydrazide ones), which made impossible getting reliably high-degree of conjugation by hyaluronidase with enzyme using carbodiimide or hydrazide conjugation method. The methods of production claimed in the present invention provide for getting hyaluronidase preparations having the degree of conjugation of not less than 90 % even on a wholesale scale.

Availability of big portions of copolymer molecule comprising only N-oxide groups makes polymer moleculeless stable. The stability study demonstrated that conjugates based on such polymer molecules with time undergo degradation. The rate of degradation of a polymer carrier with irregular distribution of N-oxide groups is not constant, the degradation occurs rather quickly during the first months of storage. On the contrary, the polymer carrier, having uniform distribution of N-oxide groups, is much more stable, its degradation rate is less and constant. The conjugate stability and uniformity of N-oxide groups distribution in the polymer carrier also depends on the conditions of the method for copolymer production for conjugation (see Table 1 and Table 2).

The claimed invention relating to the method for production of hyaluronidase conjugates provides for production of a polymer carrier having not only preset quantitative data (quantity of various units in a polymer), but the required distribution of units, which qualitatively changes the properties of both the polymer carrier proper, and a conjugate with an enzyme on its base.

The invention makes it possible to produce safe, high-efficiency and stable medicinal preparation (drug) possessing the properties of simultaneously inhibiting connective tissue hyperplasia and anti-inflammatory action, and representing a conjugate of hyaluronidase (a therapeutic enzyme taken from bovine seminal glands) and water-soluble copolymer of general formula (I).

In the case of azide method of conjugation alkylation is carried out with the use of alkyl ester of haloacetic acid, and alkylation step and hydrazinolysis are combined. The conjugation with hyaluronidase is performed by azide method at temperatures ranging from 0 to 25 °C.

Cleaning is a three-fold stepwise washing with purified water on semitransparent cassette (with lower limit of particles cutoff ranging from 1 to 30 kDa) upon finishing oxidation, alkylation and hydrazinolysis, as well as conjugation steps. Conjugation is carried out with the use of water-soluble copolymer of general formula (I) comprising in its chain from 3 to 20 % hydrazide groups.

In the case of carbodiimide method of conjugation poly-1,4-ethylene piperazine is first alkylated and then subjected to oxidation in aqueous environment. In this process alkylation is performed by using haloacetic acid.

In carbodiimide method cleaning is done in a stepwise manner by washing with purified water on semitransparent cassettes (with lower limit of particles cutoff ranging from 1 to 30 kDa) upon finishing the steps of copolymer obtaining and conjugation.

The conjugation is carried out with the use of water-soluble copolymer of N-oxide poly-1,4-ethylene piperazine of general formula (I) comprising in its chain up to 25 % of carboxyl groups, and use is made of any water-soluble carbodiimides in amount from 3 to 50 % from the copolymer involved in the reaction.

The invention subject matter is also an active conjugate ingredient possessing the properties of inhibiting hyperplasia of connective tissue and anti-inflammatory action, and being produced by any claimed methods for preparation of a medicinal agent in a form of administration selected from a suppository, ointment, injection, or cosmetic cream, including drugs for veterinary. Given below is the detailed description of the claimed method with the use of azide and carbodiimide methods of conjugation of production of Longidaza having particular properties described in detail in 10 Examples and presented in Table 1.

Under conditions of claimed technology of polymer carrier and hyaluronidase conjugates production, as it was found, there occurs the desired distribution of functional units in the copolymer structure, wherein polymer carrier and

conjugates on its base become resistant in the process of long-term storage. Said result is achieved by performing the reaction of polyethylene piperazine oxidation under dissociating conditions by way of adding urea. At that, non-covalent intermolecular bonds of polyethylene piperazine get destroyed, the reactions involve separate polymer molecules, rather than molecules in the composition of a micelle, the desired uniform distribution of functional groups along the polymer chain is achieved, there get increased enzyme activity (by 30 to 65 %, see Table 1), the conjugation degree and target product yield (by 15 to 20 %, see Table 1), and stability of hyaluronidase conjugate. Enzyme activity of Longidaza preparations kept in storage at a temperature of 2 to 8 °C within 1 year dropped by 4 to 6 % for the specimens obtained with the use of urea, and by 24 to -27 % without use of urea (Table 1, Example 4 and Example 9).

The conjugate is produced by the following two methods:

the method for hyaluronidase conjugate production using the azide method of conjugation; and
the method for hyaluronidase conjugate production using the carbodiimide method of conjugation.

Poly-1,4-ethylene piperazine (PEP) with molecular weight from 20 to 60 kDa is used as a feedstock for all methods of production.
PEP results from cationic polymerization, which is an efficient method of synthesis of monodisperse high-molecular compounds having preset molecular weight and structure,

1. Method for production of conjugate using azide method of conjugation.

This production method consists of the basic steps of the process, namely, oxidation in the presence of urea, alkylation, hydrazinolysis, conduct of conjugation using azide method,

Oxidation of poly-1,4-ethylene piperazine is carried out in an acidic medium by means of hydrogen peroxide in the presence of urea at a temperature of 45 to 55 °C for 12 to 24 hours in a water-jacketed reaction vessel. Then, the reaction feed is diluted, filtered in a cartridge filter provided with a filtering element having a pore-size of 0.45 nm, undergoes diafiltration in an ultrafiltration unit with cassettes having the lower limit of particles cutoff of 5 kDa, and gets filtered in a cartridge filter provided with a filtering element having a pore-size of 0.22 nM. Thereafter, the resultant product is dried, and tested for compliance with the regulatory document requirements and turn in for storage.

At the second step N-oxide poly-1,4-ethylene piperazine undergoes alkylation by means of ester of haloacetic acid in a water-organic solution (a mixture of water and N-methyl formamide) with agitation within a temperature range of 35 to 50 °C for 4 to 6 hours, and then, hydrazinolysis of alkyl derivative of polyethylene piperazine is done by processing using hydrazine hydrate at temperatures of 2 to 8 °C. The reaction feed is filtered in a cartridge filter provided with a filtering element having a pore-size of 0.45 nm, then it undergoes cleaning in an ultrafiltration unit with cassettes having the lower limit of particles cutoff of 10 kDa (percentage of hydrazine hydrate not exceeding 0.001%), thereafter the feed is subjected to sterilizing filtration in a cartridge filter and reconstituted in glass vials. Finally, it is tested for compliance with the requirements of the regulatory document and turned in for storage.

At the last third step the process of conjugation of hyaluronidase with hydrazide compound of carboxymethyl-containing N-oxide polyethylene piperazine comprising in its chain more than 3 % of hydrazide groups is performed using azide method by treatment with sodium nitrite at pH = 0-1 and subsequent conjugation of azide derivate with hyaluronidase for 18 to 22 hours at indoor temperature. The hyaluronidase conjugate is filtered, cleaned by washing in an ultrafiltration unit (cassettes with the lower limit of particles cutoff of 5 kDa), filter-sterilized and freeze dried (lyophilized). Test results are shown in Table 1 **(Examples 1-3).**

The claimed production method, which includes the steps of alkylation, oxidation (but without urea addition), hydrazinolysis, conjugation by azide method and cleaning, is used to obtain a Longidaza specimen. The results of this specimen testing are presented in Table 1 **(Example 4).** The comparative studies demonstrate that addition of urea increases enzyme activity, degree of conjugation and yield of target product, and conjugate stability (Table 1).

The derived freeze-dried (lyophilized) product is reconstituted in glass vials and turned in for storage in the form of an active conjugate ingredient having the activity similar to that of Longidaza®.
Further on, the obtained active pharmaceutical ingredient is used for production of liquid or freeze-dried injectable dosage form. In this regard the solution is diluted to ensure that in 1 ml of solutiona the enzyme activity is 3,000 IU (therapeutic dose), and it is tested in all parameters for compliance with the requirements of the regulatory document for finished-dosage form. After that the solution can be freeze-dried (lyophilized medicinal preparation) or, without drying, the liquid medicinal preparation get packaged, labeled and tested in all parameters for compliance with the requirements set forth in the regulatory document for finished-dosage form.

The carbodiimide method, claimed in the present invention, makes possible the process performance without use of organic solvents and harmful, the end product, i.e., the hyaluronidase conjugate with derivatives of polyethylene piperazin, improvement and stability.

2. Method of Longidaza production with the use of carbodiimide method of conjugation.

The sequential production process consists of the basic steps, namely, alkylation, oxidation in the presence of urea, conduct of conjugation using carbodiimide method.

There is used a poly-1,4-ethylene piperazine with preset properties (see above), which is alkylated by bromoacetic acid in aqueous environment at a temperature of 40 to 95 °C and weight ratio (85 : 15) - (70 : 30), respectively. The feed is mixed at a temperature of 50 to 70 °C for 3 hours, then urea, acetic acid and hydrogen peroxide are added to the reaction mass, and thereafter the resultant mixture is kept, being continuously stirred, for 18 to 22 hours. Then, the oxidation process completeness is checked with the aid of ammonia test (similar to Example 1). The reaction mass is filtered in a cartridge filter provided with a filtering element having a pore-size of 0.45 nm. The polymer is cleaned by washing in an ultrafiltration unit with the lower limit of particles cutoff of 5 kDa to a hydrogen peroxide content of not more than 0.001% (the hydrogen peroxide content test). The solution is concentrated to 10-15 %, the content of carboxyl groups in the polymer shall not be less than 6%. The calculated amount of hyaluronidase is introduced into the reaction medium, while stirring, and the medium pH is brought up to a value of 4.8.

With stirring being continued, a water-soluble carbodiimide is added to ensure that its content is 25 to 50 % of added hyaluronidase (expressed as protein). The reaction feed is kept at a temperature of 0 to 25 °C for 1 to 1.5 hours, and then there is checked the degree of conjugation which shall be not less than 95 %. In case of a positive result the reaction feed is washed with purified water, alkalified to pH = 6.8 to 7.0 and filtered in disc filter equipped with AP-15 deep-bed filter. After prefiltration the reaction feed cleaning is continuing by washing with purified water on a Pellicon-cassette to ensure that the total amount of water would be at least 200 liters per 1 kg of dry matter. Then, the solution ingredient having the Longidaza® activity is concentrated, sterile filtered in a cartridge filter fitted with a filtering membrane with a pore-size of 0.22 nm and reconstituted in glass vials in the form of a liquid ingredient. The resultant sterile masterbatch can also be freeze-dried sterile with obtaining in this case an ingredient having the activity similar to that of Longidaza® preparation in dry state. Thereafter it is checked for compliance with the requirements of the regulatory document and turned in for storage as an end product. The ingredient test results are given in Table 1 (Examples 6 to 8).

The claimed production method, which includes the steps of alkylation, oxidation, but without urea addition, conjugation by carbodiimide method and cleaning procedure, was used to get the Longidaza specimen, the test results of which are presented in Table 1 (Example 9). In the same way as for the case of the Longidaza production with the use of azide method of conjugation, urea addition increases enzyme activity, conjugation degree and target product yield, and the conjugate stability (Table 1, Table 2).

Sterile solution of the hyaluronidase conjugate is used for the intended purpose. The resultant active pharmaceutical ingredient is used for preparation of liquid or freeze-dried injectable dosage forms. With this in view this solution is diluted to ensure that in 1 ml of solution the enzyme activity is 3,000 IU (therapeutic dose) and reconstituted in glass vials and freeze-dried (lyophilized medicinal preparation) or, without drying, the liquid medicinal preparation get packaged, labeled and tested in all parameters for compliance with the requirements set forth in the regulatory document for finished-dosage form.

In the process of hyaluronidase conjugate with polyethylene piperazine derivatives the ultrafiltration cleaning on cassettes with particles cutoff of 1 to 30 kDa is used to remove process impurities. Washing is done on completion of alkylation and oxidation steps, and at the end of the production process. The claimed carbodiimide method of conjugation is carried out exclusively in aqueous environment.

Thus, the essence of the claimed method consists in a new approach in the technology of production of immobilized compounds on a polymer carrier of new class in the form of water-soluble PEP derivatives as per azide and carbodiimide methods. Copolymer is obtained by chemical modification from PEP in the presence of urea, which in the process of conjugation significantly boosts the hyaluronidase activity, degree of conjugation, yield of preparation having the Longidaza activity and its stability in storage.

Analysis of results of production of hyaluronidase conjugate with derivatives of polyethylene piperazine are presented in Table 1 (Examples 1 thru 10) indicates high yield of the target product. At that, the process with the use of carbodiimide method provides for higher degree of conjugation and end product activity, and the process is carried out exclusively in aqueous environment.

The inventive step of developed methods of production of hyaluronidase conjugate with derivatives of polyethylene piperazine is verified by absence in this business profile of continuous processes, and by the fact that production of N-oxide polyethylene piperazine was carried out with the use of urea. In industrial setting the carbodiimide method is more preferable than the azide method. The employment of developed methods in production of long-acting forms of compounds of various applications improves the production of a new preparation having the activity higher than the activity of Longidaza® preparation.

The studies of decomposition of copolymers of N-oxide 1,4-ethylene piperazin, (N-carboxymethyl)-1,4-ethylene

piperazin and 1,4-ethylene piperazin have shown that at a temperature not above 70 °C in aqueous environment with pH not exceeding 9, decomposition occurs exclusively over N-oxide units. Thermal decomposition of N-oxides proceeds by way of Meisenheimer rearrangement.

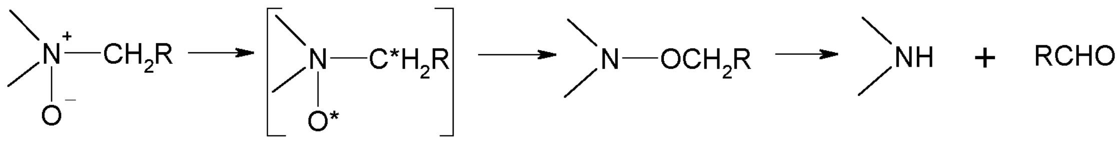

Copolymer having uniform distribution of various units should not have big portions consisting of units of only one type. Thus, with N-oxide units content of 50 % and more and their uniform distribution along the polymer chain, the copolymer shall not have big portions composed of only unsubstituted units of 1,4-ethylene piperazin (units 1). In light of this, there has been developed a method of qualitative and quantitative evaluation of uniformity of distribution of units comprising N-oxide groups along the copolymer chain. The method consists in the copolymer controlled decomposition with subsequent physic-chemical analysis of resultant fragments. It was found that with urea use in the process of oxidation the decomposition products do not contain low-molecular polymer comprising one-type units:

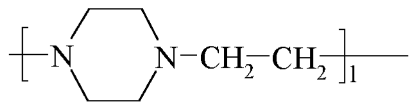

where I = 3 and above.

Availability of this substance in the copolymer decomposition products is indicative of significant non-uniformity of N-oxide units distribution in starting copolymer.
When analyzing copolymer of series 111114 using the degradation method the following substance was extracted

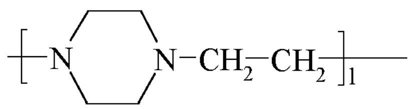

where 1 = from 4 to 6.

Upon completion of quantitative and qualitative analysis of composition of this substance one can judge on the qualitative and quantitative profile of N-oxide groups distribution in the copolymer under study. However, the developed measuring procedure is time consuming (the monitored degradation of N-oxide groups of a copolymer takes 5 weeks) and big amount of the substance to be analyzed. With this in view, there was designed a qualitative method for rapid assessment of units distribution uniformity.

The analysis of various specimens of copolymers using the DSC (Differential Scanning Calorimetry) method it was demonstrated that the polymers of this type vigorously degrade at a temperature of about 160 °C with heat liberation. At this, various forms of peak exotherm of decomposition of specimens produced with or without urea added. Thus, there were analyzed two specimens of a polymer carrier having similar quantitative parameters, namely, molecular weight, molecular-weight distribution, number of carboxyl and N-oxide groups produced with the use of urea at the oxidation step and without urea addition. In this case, the conjugates, obtained on one polymer carrier, were stable, and those obtained on another carrier were not stable.

The DSC curves for polymers are shown in Figures 1 and 2.
The DSC curves (Figures 1 and 2) demonstrate significant difference of ratio of peak intensities within a temperature range of 150 to 170 °C. The obtained results indicate the relation between the polymer structure (units distribution profile) and its thermal stability. The received results were verified by analysis of other carriers, and they demonstrate a possibility of distinguishing the specimens of polymer carriers by different stability due to functional groups arrangement in molecules of a polymer carrier.

Given below are the particular parameters accumulated in Table 1 and presented in **Examples 1-3 and 6-8.** Table 1 also presents the results of wholesale scale series **(Example 5** and **Example 10).** Examples **4 and 9** contain the data verifying the advantages of the method of production in the presence of urea.

**Example 1.**

The method of production of hyaluronidase conjugate with derivatives of polyethylene piperazine using azide method of conjugation.

Taken are 200 grams of, preliminary chopped and cleaned by a method of quadruple washing with purified water, chips of poly-1,4-ethylene piperazine with a molecular weight of 50 kDa. Chips are oxidized in a mixture of 50 ml of acetic acid, 140 ml of 30% solution of hydrogen peroxide in water, in the presence of 50 grams of urea. Water is added in such a way that the PEP content is 15 wt. %, i.e. the volume of reaction medium should be 1.5 liters. The reaction feed is heated up to 50 °C and, being continuously stirred, is kept for 24 hours. The oxidation completeness is checked with the aid of the ammonia tesing. This testing consists in the following: 10 ml of 25% solution of ammonia are added to 1 ml of reaction feed. Absence of turbidity in solution, after keeping it for 20 minutes, is considered as a positive result. In case of the positive result the reaction feed is diluted, filtered in a cartridge filter fit with a filtering element with a pore-size of 0.45 nm, cleaned and concentrated in an ultrafiltration unit with cassettes having the lower limit of particles cutoff of 5 kDa, and filtered in a cartridge filter equipped with a filtering element with a pore-size of 0.22 nm. Thereafter, it is freeze-dried, and 204 grams of N-oxide poly-1,4-ethylene piperazine are obtained in the form of lyophilizate, which is checked for compliance with the requirements of the regulatory document and turned in for storage.

The resultant N-oxide poly-1,4-ethylene piperazine is dissolved in 1,500 ml of distilled water, and 6,000 ml of N-methyl formamide is added. The resultant solution is added with 275 ml of bromalkane ester and, being continuously stirred, is allowed to stand at temperatures of 35-50 °C for 4 to 6 hours. Then the reaction mass is cooled down to 2 - 8 °C and, being stirred, added with 250 ml of hydrazine hydrate. The reaction feed is diluted 10 times with purified water, filtered in a cartridge filter fit with a filtering element with a pore-size of 0.45 nm, and cleaned by washing on Pellicon-cassettes (with the lower limit of particles cutoff of 10 kDa) to a content of trace amount of hydrazine hydrate (not more than 0.001 %).. Further the reaction feed is concentrated to 10 - 12 % (as per target substance) sterile filtered and reconstituted in glass vials. There are obtained 2,200 ml of solution of hydrazide derivative of N-oxide poly-1,4-ethylene piperazine, which is analyzed for compliance with the requirements of the regulatory document, and turned in for storage.

The solution obtained during the previous step is cooled down to 2 to 6 °C and, being stirred and cooled, it is added with hydrochloric acid **to pH= 0-1.** Then, with stirring and cooling continued, it is added batchwise with 360 grams of sodium nitrite and the reaction mass is allowed to stand for 1.5 hours. After that the reaction medium is rendered alkaline by moist-free ash **to pH= 6.8 - 7 and, at a temperature not exceeding 10 °C,** while stirring 10% solution, containing 65 grams of hyaluronidase preparation (70 % of protein), is added. The reaction feed remains stirred for 1.5 hours, then, a sample is taken to check the conjugation degree. In the case of positive result of the conjugation degree checking (not less than 70 %) the reaction mass is filtered in a disc filter fit with a deep-bed filter, AP-15 type, and washed in 50 liters of purified water in an ultrafiltration unit with cassettes having the lower limit of particles cutoff of 5 kDa. The solution is concentrated, sterile filtered and filled in sterile glass vials (liquid ingredient). Sterile solution is also freeze-dried and packed in sterile glass vials (freeze-dried ingredient). There are obtained 231 grams of ingredient, 86% yield. The results of testing the obtained conjugate ingredient are presented in B Table 1 **(Example 1).** The resultant ingredient is used for preparation a finished medicinal product with a type of activity being identical to that of known "Longidaza preparation in the form of vaginal and rectal suppository of 3,000 IU".

**Example 2**

The procedure is similar to that of Example 1, with the difference that there is used chopped and purified PEP in amount of 200 g with a molecular weight of 40 kDa, and that 60 grams of urea are used at the oxidation step. There are obtained 227 grams of ingredient with a type of activity being identical to the activity of known Longidaza preparation in the form of lyophilizate, 84% yield. The analysis results are presented in Table 1, Example 2.

The resultant ingredient is used for preparation of cream and ointments for external use, 1000 IU.

**Example 3**

The procedure is similar to that of Example 1, with the difference that there is used chopped and purified PEP in amount of 200 g with a molecular weight of 55 kDa, and that 80 grams of urea are used at the oxidation step. There are obtained 235 grams of ingredient with a type of activity being identical to the activity of known Longidaza preparation in the form of lyophilizate, 87% yield. The analysis results are presented in Table 1, Example 3.

This Ingredient is used for preparation of injectable dosage form.

**Example 4 (Reference Example)**

The procedure is similar to that of Example 1, with the difference that urea is not added at the oxidation step.

The resultant ingredient amounting to 215 grams has the activity of Longidaza® in the form of lyophilizate, 74 % yield. The results of Longidaza® testing are given in Table 1, Example 4.

The ingredient having the activity being identical with the activity of known drug "Longidaza" is used for preparation of injectable dosage form.

**Example 5**

The procedure is similar to that of Example 1, with the difference that there is used chopped and purified PEP in amount of 4,000 g with a molecular weight of 45 kDa, and that 2,000 grams of urea are used at the oxidation step. There are obtained 4,700 grams of ingredient with a type of activity identical to the activity of known Longidaza® preparation in the form of lyophilizate, 87% yield. The test results are presented in Table 1 (Example 5).

Batching up the required amount of produced ingredient in suppository, ointment or cream bases we get respectively suppositories, creams, ointments comprising active ingredient. It is also used for preparation of injectable dosage form.

**Example 6.**

The method with the use of carbodiimide method of conjugation.

Poly-1,4-ethylene piperazine in amount of 200 g with a molecular mass of 30 kDa is added to 1.2 liters of boiling-water solution comprising 58 grams of bromoacetic acid, and keep heating it at a temperature of 70 °C for 3 hours, continuously stirring said solution. Then, the reaction mass is added with 40 ml of acetic acid, 140 ml of 30% water solutiona of hydrogen peroxide, 50 grams of urea and all are stirred at a temperature of 38-42 °C for 18 to 20 hours. Further, the oxidation state completeness is checked with the aid of ammonia test (this test is described above). In the case of a positive result the reaction feed is diluted to a concentration of 1 to 2 %, and filtered in a cartridge filter fit with a filtering element having a pore-size of 0.45 nm, cleaned by washing by purified water on a Pellicon-cassette having the lower limit of particles cutoff equal to 5 kDa. In the process of washing the solution is rendered alkaline to a value of pH = 11.0 - 11.5 with the purpose of complete cleaning to remove traces of lowmolecular organic acids. The solution cleaning is continued until the hydrogen peroxide content becomes not more than 0.001 % (hydrogen peroxide test), and the solution is concentrated to 10 % of the target substance. The resultant solution is acidified by hydrochloric acid to **pH= 4.8 - 4.9,** after that 50 grams of hyaluronidase are added. The pH value is subjected to adjustment; it should be 4.8 to 4.9. The reaction feed, being stirred, is added with a solution comprising 2.1 grams of N-(3-dimethylaminopropyl)-N'-ethyl carbodiimide hydrochloride in 210 ml of water for 1.5 to 2 hours. The conjugation reaction is carried out at a temperature of 2 to 25 °C. After adding the last portion of condensing agent solution the reaction mass is allowed to stand< being stirred, for 30 minutes at a preset temperature, and a sample is taken to determine the conjugation degree. In the case of a positive result conjugation degree should be at least 90 %, the reaction mass is allowed to stand for 18 to 20 hours, it is rendered alkaline by means of moist-free ash to a value of pH = 6.8 to 7.0, washed and filtered in a disc filter with a filtering material in the form of a deep-bed filter, AP-15 type. Then, the reaction mass is cleaned by purified water in an ultrafiltration unit on a Pellicon-cassette with the lower limit of particles cutoff equal to 5 kDa. The cleaned solution is concentrated to a content of 8-10 %, and sterile filtered in a cartridge filter fit with a filtering element having a pore-size of 0.22 nm. The resultant solution of hyaluronidase conjugate with derivatives of polyethylene piperazine can be diluted to a concentration containing in one milliliter of solution 3,000 IU of enzyme activity, and finally get an injectable dosage form (in liquid form) or freeze-dried (in lyophilized form). In the case of freeze-drying, 261 grams of ingredient are obtained with a type of its activity being identical to the activity of known Longidaza® preparation, 97% yield. The test results are presented in Table 1, Example 6.

Batching up the required amount of produced ingredient in suppository base we get respectively suppositories.

**Example 7**

The procedure is similar to that of Example 6, with the difference that there is used chopped and purified PEP in amount of 200 g with a molecular weight of 38 kDa, and that 60 grams of urea are used at the oxidation step. There are obtained 265 grams of ingredient with a type of activity identical to the activity of known Longidaza® preparation in the form of lyophilizate, 98% yield. Test results are presented in Table 1, Example 7.

This ingredient is used for preparation of an ointment.

**Example 8**

The procedure is similar to that of Example 6, with the difference that there is used chopped and purified PEP in amount of 200 g with a molecular weight of 26 kDa, and that 70 grams of urea are used at the oxidation step. There

are obtained 264 grams of ingredient with a type of activity identical to the activity of known Longidaza® preparation in the form of lyophilizate, 98% yield. Test results are presented in Table 1, Example 8.

This ingredient is used for preparation of injectable dosage form.

**Example 9 (Reference Example)**

The procedure is similar to that of Example 6, with the difference that there is used chopped and purified PEP in amount of 200 g with a molecular weight of 26 kDa, and that at the oxidation step no urea is added. There are obtained 217 grams of the ingredient with a type of activity identical to that one of the known Longidaza preparation in the form of lyophilizate, 82% yield. The test results are presented in Table 1, Example 9.

The ingredient is used for preparation of injectable dosage form comprising Longidaza.

**Example 10**

The procedure is similar to that of Example 6, with the difference that there is used chopped and purified PEP in amount of 4,000 g with a molecular weight of 36 kDa, and that at the oxidation step 1,500 grams of urea is added. There are obtained 5,130 grams of the ingredient with the type of activity being identical to that of known Longidaza preparation in the form of lyophilizate, 95% yield. The test results are presented in Table 1 (Example 10).

Batching up the required amount of produced ingredient in suppository, ointment or cream bases we get respectively suppositories, creams, ointments comprising active ingredient. It is also used for preparation of injectable dosage form.

The claimed continuous carbodiimide method makes it possible to exclude from the process organic solvents, to simplify the process, upgrade the quality and increase the target product yield, to achieve high value of the conjugation degree, increase the stability of Longidaza® (in freeze-dried and liquid form) under its exposure to various environmental effect in the process of its processing and storage.

The claimed methods provide for improvement of processes of production of long-acting conjugates based on the new class carriers of compounds in the form of water-soluble derivatives of heterocyclic aliphatic aminopolymer N-oxides, comprising in their chain azide or carboxyl groups, and non-stable compounds of protein or other nature comprising active amine groups in its composition. The method has great practical importance in providing for the pharmaceutical production long-acting medicinal preparations being in great demand at the market of medicinal preparations, and it can also be used for preparation of veterinary medicines. In perspective, the developed production processes can be widely used for solving great many persistent problems in various sectors of national economy of the country as a whole.

Table 1. Results of analytical monitoring of series of hyaluronidase conjugate with copolymer of N-oxide 1,4-ethylene piperazine and (N-carboxymethyl)-1,4-ethylene piperazinium halogenide derived in EXAMPLES 1 through 10.

| Method of production | Enzyme activity at the time of production, IU/mg of drug | Enzyme activity after 1 year of storage *), IU/mg of drug | Enzyme activity decrease within 1 year, % | Conjugation degree, % | Target product yield, % | Production of medicinal preparation with Longidaza |
|---|---|---|---|---|---|---|
| EXAMPLE 1 | 221 | 212 | 4 | 85 | 86 | Suppositories |
| EXAMPLE 2 | 230 | 219 | 5 | 84 | 84 | Cream, ointment |
| EXAMPLE 3 | 242 | 230 | 5 | 86 | 87 | Injections |
| EXAMPLE 4 w/o urea | 168 | 123 | 27 | 65 | 74 | Injections |
| EXAMPLE 5 industrial batch 4, kg | 235 | 221 | 6 | 85 | 87 | Suppositories, injections, cream, ointment |
| EXAMPLE 6 | 265 | 254 | 4 | 96 | 97 | Suppositories |

(continued)

| Method of production | Enzyme activity at the time of production, IU/mg of drug | Enzyme activity after 1 year of storage *), IU/mg of drug | Enzyme activity decrease within 1 year, % | Conjugation degree, % | Target product yield, % | Production of medicinal preparation with Longidaza |
|---|---|---|---|---|---|---|
| EXAMPLE 7 | 280 | 266 | 5 | 99 | 98 | Cream |
| EXAMPLE 8 | 275 | 264 | 4 | 99 | 98 | Injections |
| EXAMPLE 9 w/o urea | 175 | 133 | 24 | 85 | 82 | Injections |
| EXAMPLE 10 industrial batch 4, kg | 289 | 272 | 6 | 97 | 95 | Suppositories, injections, cream, ointment |
| *) Storage at t = 2 to 8 °C. | | | | | | |

Table 2. Study of stability during long-term storage of Longidaza produced with urea use (EXAMPLE 3 и EXAMPLE 8) and without urea use (EXAMPLE 9) as per 'cell-free protein' (Protein S) parameter

| Method of production | Cell-free protein, % | | | | |
|---|---|---|---|---|---|
| | at the time of production | 3 months | 6 months | 9 months | 12 months |
| EXAMPLE 3 | 14 | 15 | 16 | 16 | 17 |
| EXAMPLE 8 | 1 | 1 | 2 | 2 | 3 |
| EXAMPLE 9 (w/o urea) | 15 | 26 | 30 | 32 | 33 |

## Claims

1. A method for preparation of active conjugate of hyaluronidase enzyme with a copolymer comprising N-oxide 1,4-ethylene piperazine and (N-carboxymethyl)-1,4-ethylene piperazinium halogenide with the use of azide method of conjugation, cleaning, concentrating and diluting or freeze drying, **characterized in that** conjugation is made with the use of water-soluble copolymer, which is a copolymer N-oxide 1,4-ethylene piperazine, hydrazide of (N-carboxymethyl)-1,4-ethylene piperazinium and 1,4-ethylene piperazine of general formula

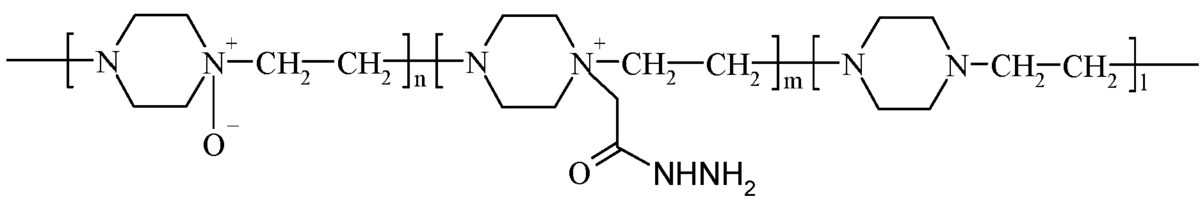

where n is ranging from 40 % to 90 % of total number of units;
m is ranging from 3 to 20 % of total number of units;

$$n + m + l = 100\ \%,$$

obtained from poly-1,4-ethylene piperazine by way of oxidation, alkylation, and hydrazinolysis, wherein the oxidation is performed by hydrogen peroxide in presence of urea, wherein alkylation is carried out by alkyl esters of haloacetic

acid, wherein the alkylation and hydrazinolysis steps are combined.

2. The method of claim 1, **characterized in that** the oxidation step is carried out with addition of 1 to 10 %, preferably 3 to 6 wt % of urea per total mass of reaction feed, including water.

3. The method of claim 1, **characterized in that** cleaning is a three-fold stepwise washing with purified water on semitransparent cassettes with lower limit of particles cutoff ranging from 1 to 30 kDa upon finishing oxidation, alkylation and hydrazinolysis, as well as conjugation steps.

4. The method of claim 1, **characterized in that** the conjugation is made with the use of water-soluble copolymer of N-oxide poly-1,4-ethylene piperazine comprising in its chain from 3 to 20 % of hydrazide groups.

5. The method of claim 1, **characterized in that** the conjugation with hyaluronidase is carried out using azide method at temperatures ranging from 0 to 25 °C.

6. A method for preparation of active conjugate of hyaluronidase enzyme with copolymer comprising N-oxide 1,4-ethylene piperazine and (N-carboxymethyl)-1,4-ethylene piperazinium halogenide with the use of carbodiimide method of conjugation, cleaning, diluting and concentrating or freeze drying, **characterized in that** the conjugation is done with the use of water-soluble copolymer, which is a copolymer N-oxide 1,4-ethylene piperazine, (N-carboxymethyl)-1,4- ethylene piperazinium and 1,4-ethylene piperazine of general formula

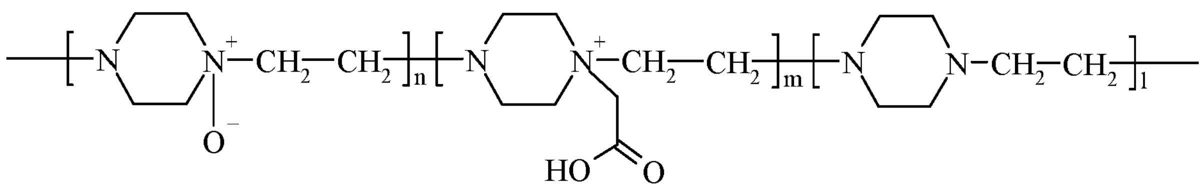

where n is ranging from 40 % to 90 % of total number of units;
m is ranging from 3 to 40 % of total number of units;

$$n + m + l = 100\ \%,$$

obtained from poly-1,4-ethylene piperazine by alkylation and oxidation in aqueous environment, wherein oxidation is carried out by hydrogen peroxide in presence of urea, wherein alkylation is carried out by using haloacetic acid.

7. The method of claim 6, **characterized in that** the oxidation step is carried out with addition of 1 to 10 %, preferably 3 to 6 wt %, of urea per total mass of reaction feed, including water.

8. The method of claim 6, **characterized in that** cleaning is carried out step-by-step using purified water on semitransparent cassettes with lower limit of particles cutoff ranging from 1 to 30 kDa upon finishing the steps of copolymer obtaining and conjugation.

9. The method of claim 6, **characterized in that** the process is carried out with the use of water-soluble copolymers of N-oxide poly-1,4-ethylene piperazine comprising in their chain up to 25 % of carboxyl groups.

10. The method of clam 6, **characterized in that** the conjugation step is carried out with the use of any water-soluble carbodiimides amounting from 3 to 50 wt % of protein added for the reaction.

11. A medicinal preparation, possessing the properties of simultaneously inhibiting connective tissue hyperplasia and anti-inflammatory action, produced using the method as described in any of claims 1 through 10 in the form of a suppository, liniment, injection or cosmetic cream.

**Patentansprüche**

1. Verfahren zur Herstellung eines aktiven Konjugats des Hyaluronidase-Enzyms mit einem Copolymer aus N-Oxid

1,4-Ethylenpiperazin und (N-Carboxymethyl)-1,4-Ethylenpiperaziniumhalogenid unter Anwendung des Azid-Verfahrens zum Konjugieren, Reinigen, Konzentrieren und Verdünnen oder Gefriertrocknen, **dadurch gekennzeichnet, dass** die Konjugation unter Verwendung eines wasserlöslichen Copolymers erfolgt, das ein Copolymer aus N-Oxid 1,4-Ethylenpiperazin, Hydrazid von (N-Carboxymethyl)-1,4-Ethylenpiperazinium und 1,4-Ethylenpiperazin in der allgemeinen Formel

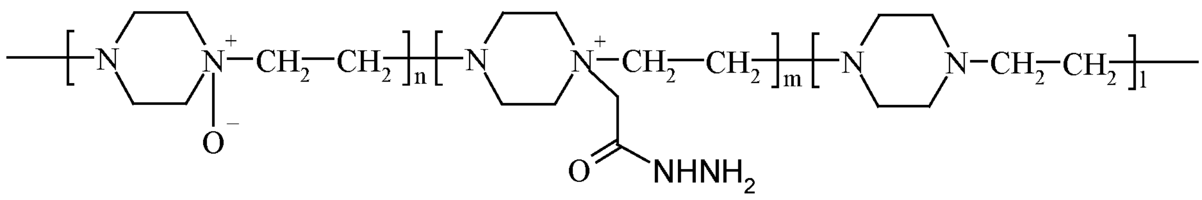

ist, wo n im Bereich von 40 % bis 90 % der Gesamtzahl der Einheiten liegt;
m von 3 bis 20 % der Gesamtzahl der Einheiten reicht;

$$n + m + l = 100\ \%,$$

und aus Poly-1,4-Ethylenpiperazin durch Oxidation, Alkylierung und Hydrazinolyse erhalten wird, worin die Oxidation durch Wasserstoffperoxid bei Anwesenheit von Harnstoff durchgeführt wird, worin die Alkylierung durch Alkylester von Halogenessigsäure erfolgt, worin die Schritte der Alkylierung und Hydrazinolyse kombiniert werden.

2. Das Verfahren nach Anspruch 1 ist **dadurch gekennzeichnet, dass** die Oxidation unter Zugabe von 1 bis 10 Gew. %, vorzugsweise 3 bis 6 Gew. % Harnstoff pro Gesamtmasse der Reaktionsbeschickung, einschließlich Wasser, durchgeführt wird.

3. Das Verfahren nach Anspruch 1 ist **dadurch gekennzeichnet, dass** die Reinigung ein dreifaches schrittweises Waschen mit gereinigtem Wasser auf halbtransparenten Kassetten mit einer unteren Grenze des Partikelausschlusses im Bereich von 1 bis 30 kDa nach Abschluss der Oxidation, Alkylierung und Hydrazinolyse sowie Konjugation umfasst.

4. Das Verfahren nach Anspruch 1 ist **dadurch gekennzeichnet, dass** die Konjugation unter Verwendung eines wasserlöslichen Copolymers von N-Oxid-Poly-1,4-Ethylenpiperazin durchgeführt wird, das in seiner Kette aus 3 bis 20 % Hydrazidgruppen besteht.

5. Das Verfahren nach Anspruch 1 ist **dadurch gekennzeichnet, dass** die Konjugation mit Hyaluronidase unter Anwendung des Azid-Verfahrens im Temperaturbereich von 0 bis 25 °C erfolgt.

6. Verfahren zur Herstellung eines aktiven Konjugats des Hyaluronidase-Enzyms mit einem Copolymer aus N-Oxid 1,4-Ethylenpiperazin und (N-Carboxymethyl)-1,4-Ethylenpiperaziniumhalogenid, unter Anwendung des Carbodiimid-Verfahrens zum Konjugieren, Reinigen, Verdünnen und Konzentrieren oder Gefriertrocknen, **dadurch gekennzeichnet, dass** die Konjugation unter Verwendung eines wasserlöslichen Copolymers erfolgt, das ein Copolymer aus N-Oxid 1,4-Ethylenpiperazin, (N-Carboxymethyl)-1,4-Ethylenpiperazinium und 1,4-Ethylenpiperazin in der allgemeinen Formel

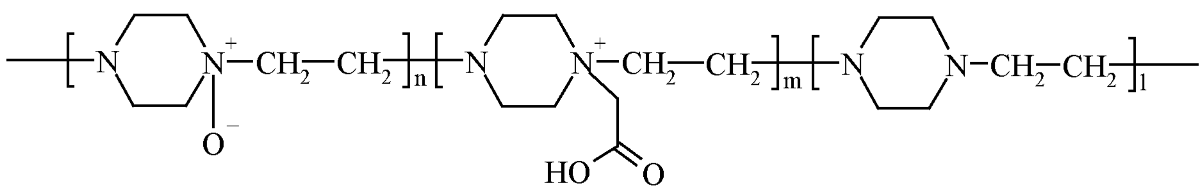

ist, wo n im Bereich von 40 % bis 90 % der Gesamtzahl der Einheiten liegt;
m von 3 bis 40 % der Gesamtstückzahl reicht;

$$n + m + l = 100\ \%$$

erhalten aus Poly-1,4-Ethylenpiperazin durch Alkylierung und Oxidation in wässriger Umgebung, worin die Oxidation durch Wasserstoffperoxid bei Anwesenheit von Harnstoff durchgeführt wird, worin die Alkylierung unter Verwendung von Halogenessigsäure erfolgt.

7. Das Verfahren nach Anspruch 6 ist **dadurch gekennzeichnet, dass** die Oxidation unter Zugabe von 1 bis 10 Gew. %, vorzugsweise 3 bis 6 Gew. % Harnstoff pro Gesamtmasse der Reaktionsbeschickung, einschließlich Wasser, durchgeführt wird.

8. Das Verfahren nach Anspruch 6 ist **dadurch gekennzeichnet, dass** die Reinigung schrittweise unter Verwendung von gereinigtem Wasser auf halbtransparenten Kassetten mit einer unteren Grenze des Partikelausschlusses im Bereich von 1 bis 30 kDa nach Abschluss der Copolymergewinnung und Konjugation durchgeführt wird.

9. Das Verfahren nach Anspruch 6 ist **dadurch gekennzeichnet, dass** das Verfahren unter Verwendung von wasserlöslichen Copolymeren von N-Oxid-Poly-1,4-Ethylenpiperazin durchgeführt wird, die in ihrer Kette aus bis zu 25 % Carboxylgruppen bestehen.

10. Das Verfahren nach Anspruch 6 ist **dadurch gekennzeichnet, dass** der Konjugationsschritt unter Verwendung von beliebigen wasserlöslichen Carbodiimiden in einer Menge von 3 bis 50 Gew. % Protein durchgeführt wird, das der Reaktion hinzugefügt wird.

11. Medizinisches Präparat mit den Eigenschaften der gleichzeitigen Hemmung der Bindegewebshyperplasie und entzündungshemmender Wirkung, in Form eines Zäpfchens, Liniments, einer Injektion oder kosmetischer Creme, nach einem in den Ansprüchen 1 bis 10 beschriebenen Verfahren, hergestellt.

## Revendications

1. Procédé pour la préparation d'un conjugué actif d'enzyme hyaluronidase avec un copolymère comprenant de la N-oxyde 1,4-éthylène pipérazine et un halogénure de (N-carboxyméthyl)-1,4-éthylène pipérazinium avec l'utilisation d'un procédé à l'azide de conjugaison, nettoyage, concentration et dilution ou lyophilisation, **caractérisé en ce que** la conjugaison est faite avec l'utilisation d'un copolymère hydrosoluble, qui est un copolymère de N-oxyde 1,4-éthylène pipérazine, d'hydrazide de (N-carboxyméthyle)-1,4-éthylène pipérazinium et de 1,4-éthylène pipérazine de formule générale

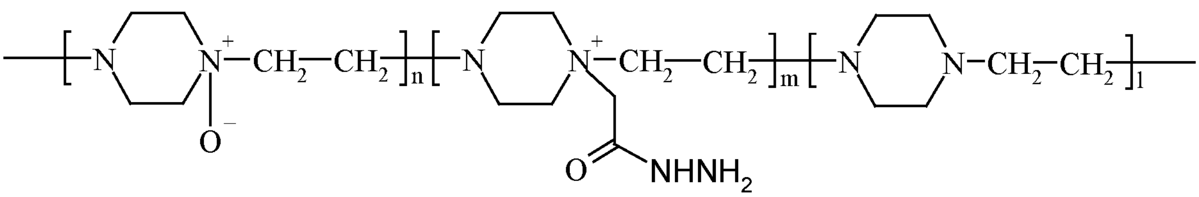

où n va de 40 % à 90 % du nombre total de motifs ;
m va de 3 à 20 % du nombre total de motifs ;

$$n + m + l = 100\ \%,$$

obtenu à partir de poly-1,4-éthylène pipérazine au moyen d'une oxydation, d'une alkylation, et d'une hydrazinolyse, dans lequel l'oxydation est mise en œuvre par un peroxyde d'hydrogène en présence d'urée, dans lequel l'alkylation est effectuée par des esters alkyliques d'acide haloacétique, dans lequel les étapes d'alkylation et d'hydrazinolyse sont combinées.

2. Procédé selon la revendication 1, **caractérisé en ce que** l'étape d'oxydation est effectuée avec l'addition de 1 à 10 %, de préférence 3 à 6 % en poids d'urée par masse totale de charge de réaction, incluant de l'eau.

**3.** Procédé selon la revendication 1, **caractérisé en ce que** le nettoyage est un lavage par paliers en trois fois avec de l'eau purifiée sur des cassettes semitransparentes avec une limite inférieure de coupure de particules allant de 1 à 30 kDa à la fin des étapes d'oxydation, d'alkylation et d'hydrazinolyse, ainsi que de conjugaison.

**4.** Procédé selon la revendication 1, **caractérisé en ce que** la conjugaison est faite avec l'utilisation de copolymère hydrosoluble de N-oxyde poly-1,4-éthylène pipérazine comprenant dans sa chaîne de 3 à 20 % de groupes hydrazide.

**5.** Procédé selon la revendication 1, **caractérisé en ce que** la conjugaison avec de l'hyaluronidase est effectuée en utilisant un procédé à l'azide à des températures allant de 0 à 25 °C.

**6.** Procédé pour la préparation de conjugué actif d'enzyme hyaluronidase avec un copolymère comprenant de la N-oxyde 1,4-éthylène pipérazine et un halogénure de (N-carboxyméthyl)-1,4-éthylène pipérazinium avec l'utilisation d'un procédé à l'azide de conjugaison, nettoyage, dilution et concentration ou lyophilisation, **caractérisé en ce que** la conjugaison est faite avec l'utilisation de copolymère hydrosoluble, qui est un copolymère de N-oxyde 1,4-éthylène pipérazine, de (N-carboxyméthyl)-1,4- éthylène pipérazinium et de 1,4-éthylène pipérazine de formule générale

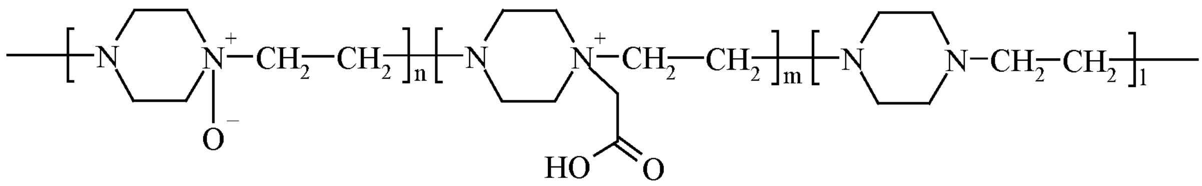

où n va de 40 % à 90 % du nombre total de motifs ;
m va de 3 à 40 % du nombre total de motifs ;

$$n + m + l = 100\ \%,$$

obtenu à partir de poly-1,4-éthylène pipérazine par alkylation et oxydation dans un environnement aqueux, dans lequel l'oxydation est effectuée par du peroxyde d'hydrogène en présence d'urée, dans lequel l'alkylation est effectuée en utilisant de l'acide haloacétique.

**7.** Procédé selon la revendication 6, **caractérisé en ce que** l'étape d'oxydation est effectuée avec l'addition de 1 à 10 %, de préférence 3 à 6 % en poids, d'urée par masse totale de charge de réaction, incluant de l'eau.

**8.** Procédé selon la revendication 6, **caractérisé en ce que** le nettoyage est effectué étape par étape en utilisant de l'eau purifiée sur des cassettes semitransparentes avec une limite inférieure de coupure de particules allant de 1 à 30 kDa à la fin des étapes d'obtention et de conjugaison de copolymère.

**9.** Procédé selon la revendication 6, **caractérisé en ce que** le processus est effectué avec l'utilisation de copolymères hydrosolubles de N-oxyde poly-1,4-éthylène pipérazine comprenant dans leur chaîne jusqu'à 25 % de groupes carboxyle.

**10.** Procédé selon la revendication 6, **caractérisé en ce que** l'étape de conjugaison est effectuée avec l'utilisation de n'importe quels carbodiimides hydrosolubles représentant de 3 à 50 % en poids de la protéine ajoutée pour la réaction.

**11.** Préparation médicinale, possédant les propriétés d'inhiber simultanément une hyperplasie de tissu conjonctif et une action anti-inflammatoire, produite en utilisant le procédé tel que décrit dans l'une quelconque des revendications 1 à 10 sous la forme d'un suppositoire, d'un liniment, d'une injection ou d'une crème cosmétique.

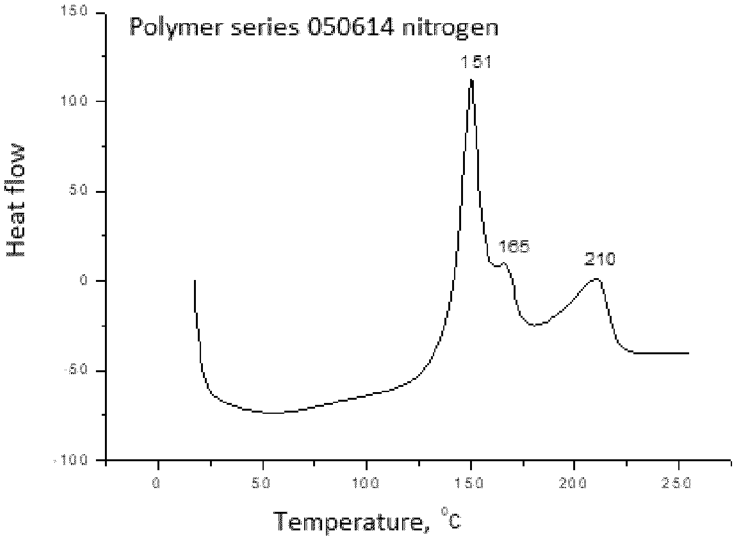
Polymer series 050614 nitrogen
151
165
210
Heat flow
Temperature, °C
150
100
50
0
-50
-100
0
50
100
150
200
250

Fig 1

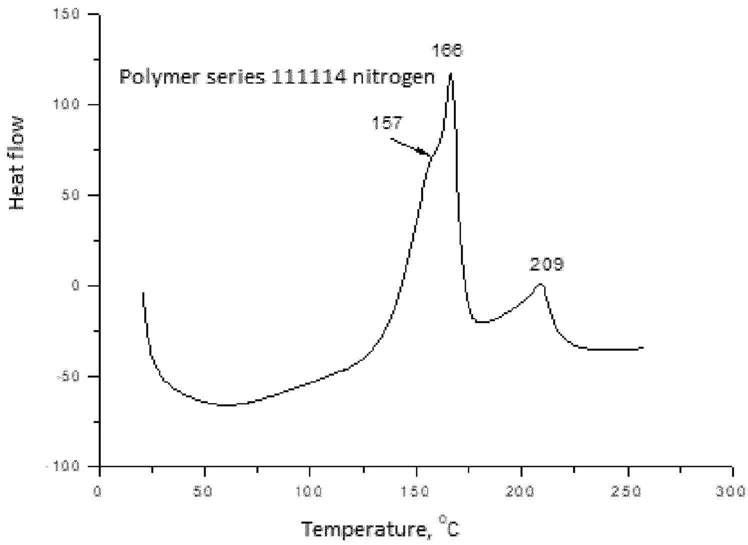
Polymer series 111114 nitrogen
166
157
209
Heat flow
Temperature, °C
150
100
50
0
-50
-100
0
50
100
150
200
250
300

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2180595, Beilon P.S., Palleroni A. B.
- RU 2232163, Burg Y, Hilger B., Josel H.-P.
- RU 2298560, Kurochkin S.N., Parkansky A.A.
- RU 2073031, Nekrasov A.V, Puchkova N.G., Ivanova A.S.
- RU 2185388
- RU 2556378
- RU 2112542


### Non-patent literature cited in the description


- **PARVEEN S ; SAHOO S.K.** *Clin Pharmacokinet,* 2006, vol. 45 (10), 965
- **DUNCAN R.** PEGylated Protein Drugs: Basic Science and Clinical Applications. Birkhäuser, 2009
- **HARRIS J.M ; CHESS RB.** Effect of pegylation on pharmaceuticals. *Nat Rev Drug Discov,* 2003, vol. 2 (3), 214
- **NEKRASOV A.V. ; PUCHKOVA N.G.** *Immunology (Immunologiya),* 2006, vol. 27 (2), 1